# EUROPEAN PATENT APPLICATION

(11) **EP 1 264 598 A1**
(43) Date of publication of application: **11.12.2002**
(21) Application number: 01202157.2
(22) Date of filing: 05.06.2001
(51) Int. Cl.: A61K 38/17, A61P 35/00, A61P 37/02, A61K 39/395

(54) **Altering an immune response by influencing the interaction of LAIR with its ligand**

(71) Applicant: Universitair Medisch Centrum Utrecht, 3584 CX Utrecht (NL); Universiteit Utrecht, 3584 CX Utrecht (NL)
(72) Inventor: The designation of the inventor has not yet been filed
(74) Representative: Prins, Adrianus Willem

(57) **Abstract**

The invention provides a method to modulate the interaction between a LIAR and its ligand, said ligand being Ep-CAM or a functional equivalent thereof, comprising providing Ep-CAM or a functional equivalent thereof, or providing an Ep-CAM antagonist. Preferably, said LAIR is part of an immune cell. With a method of the invention it is possible to modulate LAIR-induced "silencing" of immune cells. In one aspect the invention provides a compound capable of at least in part reducing the LAIR-induced "silencing" of immune cells.

Use of an Ep-CAM, an Ep-CAM antagonist, and/or a functional equivalent thereof for modulating the LAIR-induced "silencing" of immune cells is also provided.

## Description

The invention relates to the field of immunology. More specifically the invention relates to methods to modulate an immune response.

In the last decades, the significant role of the mammalian immune system in combating a broad variety of diseases has been extensively studied. An immune response involves a complex combined action of a lot of components. The actions of said components are controlled in many ways. For instance, an immune response can be enhanced by a cascade of reactions leading to an enhanced activity of immune cells towards a specific antigen. Likewise, an immune response can be at least partly inhibited in many ways. Inhibitory receptors, bearing immunoreceptor tyrosine-based inhibitory motifs (ITIMs), play a pivotal role in balancing the immune response (reviewed in (1). Rather than relaying activation signals, these receptors suppress cellular functions. One or more ITIMs in the cytoplasmic tail are responsible for the inhibitory signal upon ligation of the receptors by their ligand. ITIMs are short amino acid motifs with the consensus sequence I/V/L/SxYxxL/V, which are able to recruit cytoplasmic protein tyrosine phosphatases upon phosphorylation of the central tyrosine residue. These phosphatases abrogate signaling through activating receptors, thereby preventing cellular immune functions such as cytotoxicity or proliferation. In recent years, many novel inhibitory receptors have been identified and recognized to be necessary for the immune system to prevent excessive activation or autoimmunity.

The Leukocyte Associated Immunoglobulin-like Receptor-1 (LAIR-1) (2) is a member of the Ig superfamily that is expressed on the majority of peripheral blood mononuclear cells, including NK cells, T cells, B cells, monocytes and dendritic cells, as well as the majority of thymocytes. Cross-linking of LAIR-1 by mAb *in vitro* delivers a potent inhibitory signal that is capable of inhibiting cellular functions of NK cells, effector T cells, B cells and dendritic cell precursors (2,3,4,5). In addition to LAIR-1, we identified LAIR-2, a putative secreted protein that is 84% homologous to LAIR-1 (3).

LAIR-1 has two ITIM motifs and is structurally related to human Killer cell Ig-like Receptors (KIRs) and the immunoglobulin-like transcripts (ILTs/LIRs). The KIRs and some of the ILTs recognize MHC-Class I and are thought to play a role in the prevention of auto-immunity (reviewed in (6)). Many of the ITIM-bearing inhibitory receptors are members of multigene families that contain genes encoding activating receptors. The activating isoforms are characterized by having short cytoplasmic domains and basic amino acids within their transmembrane regions. These receptors signal through their association with immunoreceptor tyrosine-based activating motif (ITAM)-bearing transmembrane adaptor molecules, such as FcεRIχ or DAP12 (7,8).

Within the family of ITIM-bearing receptors, LAIR-1 is unique because it is extremely broadly expressed and does not recognize MHC-Class I (2). Furthermore, sequencing of the genomic region where the *LAIR* genes are located suggests that there are only two *LAIR* genes, neither of which is predicted to encode an activating receptor (9). To delineate the biological function of LAIR-1, identification of the natural ligand is imperative.

We here report the identification of Epithelial Cellular Adhesion Molecule (Ep-CAM) as a binding partner for LAIR. The Ep-CAM protein is a 38 kDa transmembrane glycoprotein that is expressed on the basolateral surface of all human simple, pseudo-stratified and transitional epithelia. The molecule has also been named EGP-2, GA733-2, CO17-1A, KSA, ESA, EGP40, 323/A3, Egp34, Ks1/4, usually after the antibodies that recognize this structure. Many of the antibodies that are raised against cells derived from human carcinomas are Ep-CAM specific, since it is a very immuno-dominant antigen. The molecule is abundantly and homogeneously expressed on cell surfaces of human carcinomas of various origins. Intestinal carcinomas express the highest levels of Ep-CAM, while tumors from non-epithelial origin are all Ep-CAM-negative.

Human Ep-CAM is encoded by the GA733-2 gene and has a mouse homologue which shows 86% similarity with the amino acid sequence of the human molecule. Ep-CAM is a type I transmembrane protein with a 23 aa signal peptide, a 242 aa extracellular domain, a 23 aa transmembrane region and a 26 aa highly charged intracellular anchor. The extracellular domain has 12 cysteine residues and 3 potential N-linked glycosylation sites. The molecule exists as two post-translationally modified variants, originating from a proteolytic cleavage site in the extracellular domain. The intracellular domain has no known (signaling) motifs.

Ep-CAM has been shown to function as a Ca²⁺ independent homophilic intercellular adhesion molecule and to modulate intercellular interactions in which cadherins play a role. The short (26 aa) cytoplasmic tail was proposed to contain two binding sites for α-actinin and is essential for its role as an adhesion molecule.

The physiological role proposed for Ep-CAM so far is in intercellular adhesion. Ep-CAM resembles classic adhesion molecules, although the adhesion mediated by Ep-CAM is relatively weak and its role in maintaining tissue integrity is questionable. We propose that Ep-CAM, through interaction with the inhibitory receptor LAIR-1 in normal physiology plays a role in the control of (mucosal) immune responses, possibly preventing excessive inflammatory responses in regions with high antigen exposure like the intestine.

Now that a binding partner for LAIR is known, it is possible to influence the interaction between LAIR of an immune cell with a LAIR binding partner. Said LAIR binding partner may be Ep-CAM or a functional equivalent thereof. This way it is possible to influence the immune response of said immune cell. Said immune response may be directed towards any antigen, for instance a tumor cell or a self-antigen. When an interaction of LAIR with a LAIR binding partner (Ep-CAM or functional equivalent thereof) is reduced, also the inhibitory effect is reduced, allowing for a stronger immune response. Thus modulating an interaction between a LAIR and its ligand opens the gate to modulate an immune response in a very precise and defined manner.

The interaction between LAIR and its ligand can be enhanced by providing Ep-CAM or a functional equivalent thereof. Providing Ep-CAM or a functional equivalent can mimic binding of LAIR to its ligand, thereby stimulating the inhibitory effect of LAIR on an immune cell. In that case, the action of a LAIR-containing immune cell can be decreased. This is called the "silencing" of an immune cell. Alternatively, the interaction between a LAIR and its ligand can be decreased by providing an Ep-CAM antagonist. Said antagonist can bind present Ep-CAM, thereby preventing binding of LAIR to said Ep-CAM. This way, the inhibitory effct of LAIR on an immune cell is decreased, resulting in an enhanced action of said immune cell. Said action may be directed towards any antigen.

Thus, in one aspect the present invention provides a method to modulate the interaction between a LAIR and its ligand, said ligand being Ep-CAM or a functional equivalent thereof, comprising providing Ep-CAM or a functional equivalent thereof, or providing an Ep-CAM antagonist. In a preferred embodiment, said LAIR is part of an immune cell. LAIR-1 is a member of the Ig superfamily that is expressed on the majority of peripheral blood mononuclear cells, including NK cells, T cells, B cells, monocytes, dendritic cells as well as the majority of thymocytes. Therefore, a preferred embodiment provides a method of the invention, wherein said immune cell comprises a NK cell, a T cell, a B cell, a monocyte, a dendritic cell and/or a thymocyte.

In addition to LAIR-1, we identified LAIR-2, a putatively secreted protein which is 84% homologous to LAIR-1. Thus in yet another aspect the invention provides a method according to the invention, wherein said LAIR-2 or a functional equivalent thereof is used as an Ep-CAM antagonist.

The transmembrane glycoprotein Ep-CAM contains two EGF-like repeats in its extracellular domain and has a short cytoplasmic tail. We have shown that Ep-CAM binds to LAIR through its first EGF-like domain. Thus, a preferred embodiment of the invention provides a method according to the invention, comprising modulating the interaction of the first EGF-like domain of said Ep-CAM protein or functional equivalent with said LAIR.

In terms of the invention, a functional equivalent is defined as a molecule which has the same kind of properties in kind, not necessarily in amount. For instance, a functional equivalent of an Ep-CAM is also capable of binding LAIR.

By an antagonist of a molecule is meant a compound which binds said molecule or its target. When said antagonist binds said molecule, at least one property of said molecule is counteracted. For instance, when an Ep-CAM antagonist binds an Ep-CAM molecule, it deprives said Ep-CAM molecule from the capability to bind LAIR. On the other hand the antagonist may block the LAIR-1 receptor without the receptor giving its signal.

As an Ep-CAM antagonist can be used for reducing the LAIR-induced "silencing" of immune cells, one embodiment of the invention also provides a use of an Ep-CAM antagonist for reducing the LAIR-induced "silencing" of immune cells. By reducing LAIR-induced silencing is meant herein that the overall effect of LAIR-induced silencing is decreased. This can be either performed by LAIR-induced silencing of less immune cells, and/or increment of the action of immune cells which are exposed to LAIR-induced silencing effect.

Reducing the LAIR-induced "silencing" of immune cells is particularly suitable when a certain antigen is capable of downregulating the action of a LAIR-comprising immune cell directed towards said antigen because of the presence of Ep-CAM on the target cell exposing said antigen. This is for instance often the case with (colorectal) carcinoma cells. These cells often express Ep-CAM on their surface. Said Ep-CAM downregulates LAIR-comprising immune cells directed toward said carcinoma cells. When an Ep-CAM antagonist is added to said Ep-CAM-comprising target cell comprising said antigen it deprives, at least in part, said antigen of the capability of silencing said immune cell. This way, a stronger immune response against said antigen is obtained. Thus, said Ep-CAM antagonist can be used for the preparation of a medicament. Therefore, in one aspect the invention provides a use of an Ep-CAM antagonist for the preparation of a medicament. In a particular aspect the present invention provides a use of an Ep-CAM antagonist for the preparation of a medicament against (colorectal) carcinoma.

Of course, also a compound capable of at least in part reducing the LAIR-induced "silencing" of immune cells is also herewith provided. Preferably, said compound comprises an Ep-CAM antagonist or a functional part, derivative and/or analogue thereof.

A functional part of a molecule is defined as a part which has the same kind of properties in kind, not necessarily in amount. A functional derivative of a molecule is defined as a molecule which has been altered such that the properties of said molecule are essentially the same in kind, not necessarily in amount. A derivative can be provided in many ways. A derivative of a protein can for instance be provided through conservative amino acid substitution.

A person skilled in the art is well able to generate analogous compounds of a molecule. An analogue of a protein can for instance be generated through screening of a peptide library. Such an analogue has essentially the same immunogenic properties of said molecule in kind, not necessarily in amount.

The invention also provides a use of Ep-CAM or of a functional equivalent thereof for enhancing LAIR-induced silencing. By enhancing LAIR-induced silencing is meant herein that the overall effect of LAIR-induced silencing is increased. This can be either performed by LAIR-induced silencing of more immune cells, and/or decrement of the action of immune cells. Said immune cells may already be exposed to some LAIR-induced silencing effect, although this is not necessary. Enhancing LAIR-induced silencing is particularly useful when an individual suffers from an auto-immune disease. In that case, often an immune response is raised against at least one autoantigen. Alternatively in other autoimmune diseases the immune system can be inappropriately activated. An autoantigen is defined as a normal part of the body against which no significant immune response is raised in a healthy individual. Said autoimmune disease comprises for instance rheumatoid arthritis, scleroderma and systemic lupus erythematosus (SLE). There is no medicament against said autoimmune diseases. Thus in these cases it is highly desirable to decrease the action of autoimmune cells by enhancing LAIR-induced silencing. This can be done by administration of Ep-CAM or a functional equivalent to an individual. Alternatively, a gene encoding said Ep-CAM or functional equivalent may be administered to said individual by means known by the person skilled in the art. Thus, one embodiment provides a use of Ep-CAM or of a functional equivalent thereof for the preparation of a medicament. A particular embodiment of the invention provides a use of Ep-CAM or of a functional equivalent thereof for the preparation of a medicament against an autoimmune disease.

The invention is further explained in more detail in the following examples. These examples do not limit the invention in any way. A person skilled in the art can think of many alternative ways, which are still within the scope of the present invention.

### Materials and Methods

### Cell lines.

293T cells were kindly provided by T. Kitamura. HT29 cells were obtained from American Type Culture Collection, Rockville MD.

### Antibodies.

The mouse anti-LAIR-1 mAb DX26 was described previously (2). The 8A8 (IgG1) producing hybridoma was generated by fusing the Sp2/0 myeloma cell line with splenocytes from a BALB/c mouse immunized with purified LAIR-1 protein. 323/A3 is a mouse anti-human Ep-CAM mAb (10) and UBS54 is a human anti-human Ep-CAM mAb isolated from a phage library, as described previously (11).

### Detection of LAIR-1-ligand.

A chimeric protein composed of the leader sequence and the extracellular part of LAIR-1 (amino acids 1-162) fused to the Fc region of human IgG1 was inserted into the pCDNA3.1 vector. The protein, designated LAIR-1-hIg, was produced by transient expression in 293T cells and subsequent purification by affinity chromatography on protein A sepharose columns. Cell-lines were screened for the presence of a putative LAIR-1-ligand by assaying for binding of the LAIR-1-hIg. 1x10E6 cells were incubated at RT for 30 min with 30 µl containing 5 µg LAIR-1-hIg, 1% BSA, 2% FCS and 2% normal mouse serum. Upon washing, 10 µg/ml biotin-conjugated goat-anti-human-IgG1 (Caltag laboratories) was added for 15 min at RT, followed by washing and 15 min incubation with phycoerytrin-conjugated streptavidin. Cells were assayed on a FACS Calibur with the addition of propidium iodide to exclude dead cells. As control IgG, either 2% pooled human serum (HPS) or a mouse CTLA4-hIg protein was used, both giving similar results.

### Cloning of LAIR-1-ligand

The colorectal carcinoma cell line HT29 was found to highly express LAIR-1-ligand as assayed by LAIR-1-hIg binding. A cDNA library from this cell line was constructed into the pCDNA3.0 vector using oligo-dT primed cDNA. cDNA cloning by transient transfection into 293T was performed as described (12) with modifications (13). Four independent cDNA clones were obtained and sequenced.

### Generation of Ep-CAM deletion mutants.

Deletion mutants of the human Ep-CAM cDNA were constructed by using PCR. PCR fragments of the extracellular domain of Ep-CAM were cloned in frame into a pCDNA3.1 vector with an N-terminal Ep-CAM leader sequence, a C-terminal transmembrane region and an intracellular domain of Ep-CAM, followed by a Myc epitope tag. All constructs were confirmed by nucleotide sequencing. After transfection into 293T cells, expression of the protein was checked by western blotting using an anti-Myc mAb. Membrane expression and transfection efficiency was monitored by staining of methanol-fixed transfected cells with anti-Myc mAb.

### Isolation and staining of intraepithelial lymphocytes.

Intraepithelial lymphocytes (IEL) were isolated from the colon from donors that underwent partial colon resection because of malignancies. Unaffected parts of the colon were used to isolate IEL as previously described (14). Cells were stained immediately after isolation with anti-CD3 and anti-LAIR-1 antibodies and analyzed by flow cytometry. All tissues were handled according to the guidelines of the institutional review board of the University Medical Center Utrecht on the use of human subjects in medical research.

### Immunohistology.

Human ileum sections were snap frozen in liquid nitrogen and stored at -70°C. Frozen sections (6µm) were cut, mounted on glass slides, dried at RT and fixed in 3.7% formaldehyde in PBS at RT for 10 min. The sections were washed with PBS containing 1.5% glycine and incubated with biotin conjugated UBS54 (anti-Ep-CAM) and a mixture of anti-LAIR-1 mAb DX26 and 8A8 for 1 h at RT. After washing, sections were incubated with TRITC-conjugated goat-anti mouse-Ig antibodies and FITC-conjugated streptavidin for 1 h at RT. Samples were counterstained with DAPI and mounted in Vectashield anti-fade mounting medium (Vector Laboratories, Burlingame, CA, USA).

### Results and Discussion

### Ep-CAM is a LAIR-1-ligand

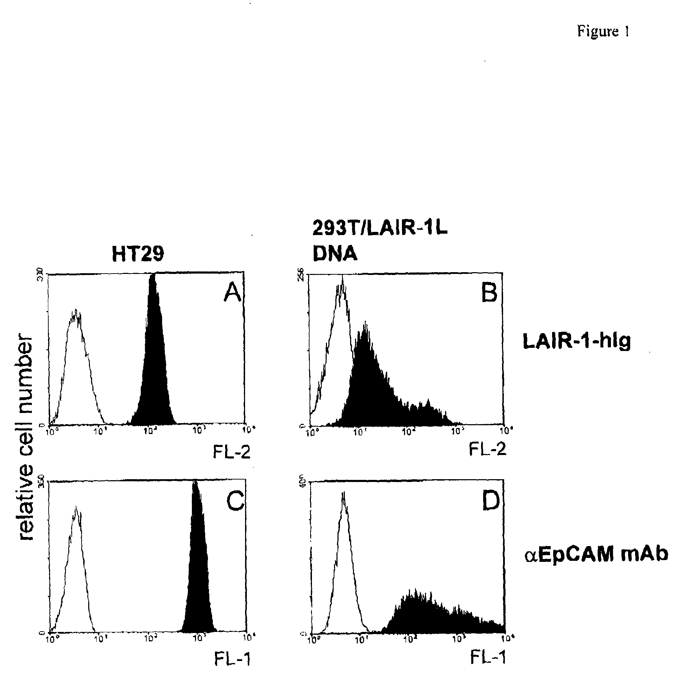

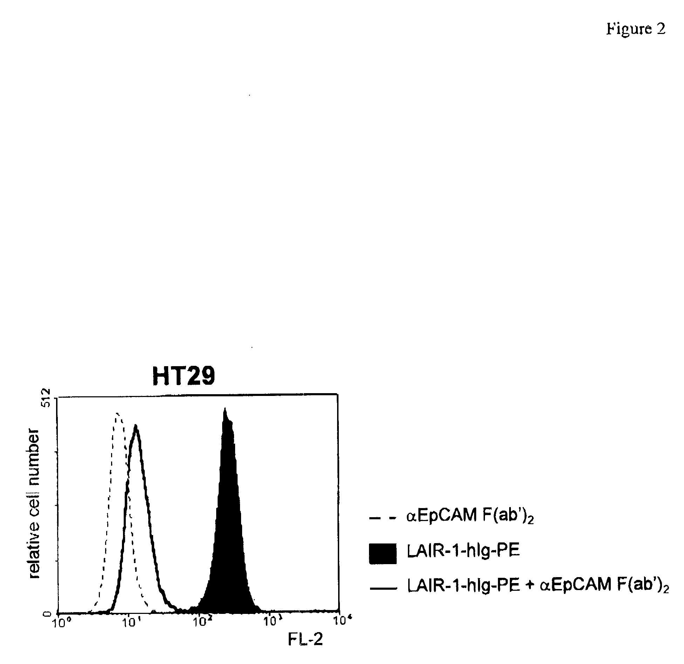

To identify the natural ligand for LAIR-1 we constructed a fusion protein of the extra-cellular domain of LAIR-1 and the Fc portion of human IgG1 (LAIR-1-hIg). This protein was used as a staining reagent to screen cell lines. Human colon carcinoma cell lines bound this fusion protein but not a control Ig fusion protein and thus expressed a putative LAIR-1-ligand (LAIR-1L). The human colon carcinoma line HT29 displayed high LAIR-1L expression (Fig. 1 A). mRNA from HT29 cells was used to construct a cDNA expression library, and expression cloning was performed. We obtained four individual cDNAs that encoded a protein (LAIR-1L) that bound the LAIR-1-hig (Fig. 1 B). Sequencing revealed that the cDNA of all four clones encoded full length Ep-CAM, (also known as EGP-2, GA733, CO17-1A, KSA, ESA, EGP40). Ep-CAM was indeed abundantly present on the surface of HT29 cells (Fig. 1 C). The identity of the protein encoded by the LAIR-1L cDNA was confirmed by staining of cells transfected with this cDNA with Ep-CAM-specific antibodies (Fig. 1 D). Binding of the LAIR-1-hIg to Ep-CAM was abolished by prior incubation with the Ep-CAM-specific mAb 323/A3, demonstrating the specificity of the interaction (Fig. 2). The LAIR-1-specific antibodies available (DX26 and 8A8) did not block the LAIR-1-hIg - Ep-CAM interaction (data not shown), indicating that these antibodies do not recognize epitopes from LAIR-1 that are involved in Ep-CAM binding.

### Ep-CAM binds both LAIR-1 and LAIR-2

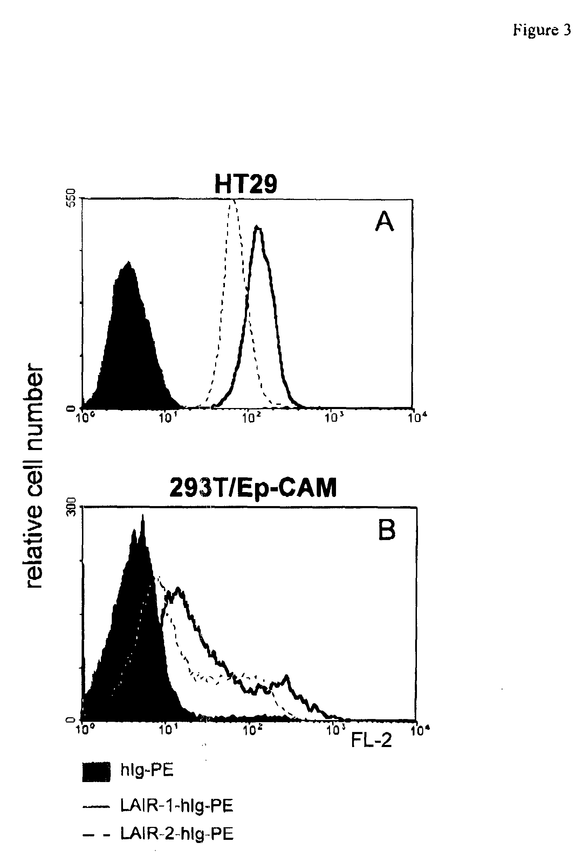

We previously described a gene highly related to LAIR-1, designated LAIR-2 (2). LAIR-2 has 84% amino acid homology in the Ig domain with LAIR-1 and cDNA for two different splice variants were identified (3). LAIR-2 is not recognized by the LAIR-1-specific mAbs (data not shown). We constructed a fusion protein of LAIR-2 with the Fc portion of human IgG, similar to the LAIR-1-hIg protein. The protein was purified in the same way as LAIR-1-hIg and used to stain HT29 cells expressing natural Ep-CAM as well as 293T cells transfected with Ep-CAM cDNA. Like LAIR-1-hIg, LAIR-2-hIg was able to bind Ep-CAM on HT29 cells (Fig. 3A), as well as Ep-CAM transfected 293T cells (Fig. 3B).

### Ep-CAM binds to LAIR through its first EGF-like domain.

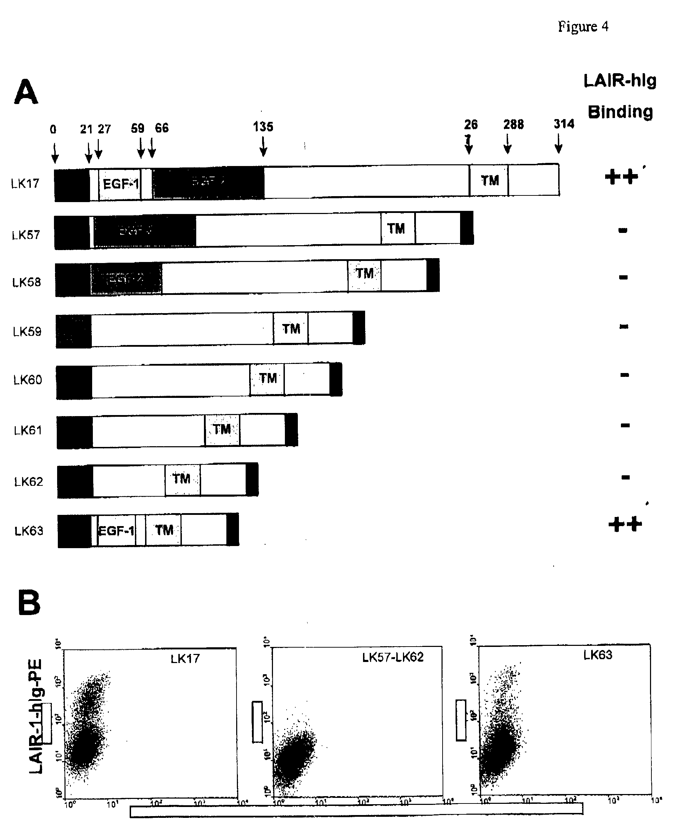

To determine which domain of Ep-CAM was responsible for binding to the LAIR molecules, deletion constructs of Ep-CAM were designed. Ep-CAM contains two EGF-like repeats in its extracellular domain and has a short cytoplasmic domain (15). Various PCR-fragments containing the different domains of the extracellular part of Ep-CAM were produced (Fig. 4 A). These were ligated into a vector containing the leader sequence of Ep-CAM at the amino-terminus and the transmembrane region and cytoplasmic tail at the carboxy terminus. All constructs were Myc-tagged at the carboxy terminus. Upon transfection in 293T, protein expression was compared by anti-Myc western blotting and transfection efficiency was assessed by staining of methanol-fixed cells with anti-Myc antibodies. Microscopic analysis of the stained cells indicated membrane expression of the proteins encoded by the various constructs and protein expression and transfection efficiency was comparable for all constructs (data not shown). The only construct, apart from full length Ep-CAM, that retained binding of LAIR-1-hIg, was a construct containing the first EGF-like repeat from Ep-CAM (Fig. 4 B). Similar results were obtained for LAIR-2-hIg (data not shown). We conclude that Ep-CAM binds to the LAIR molecules through its first EGF-like repeat.

This observation is in accordance with the finding that the anti-Ep-CAM mAb 323/A3 was able to block Ep-CAM/LAIR interaction. The epitope for this mAb maps to the first EGF-like domain of Ep-CAM (15).

### Intraepithelial lymphocytes express LAIR-1

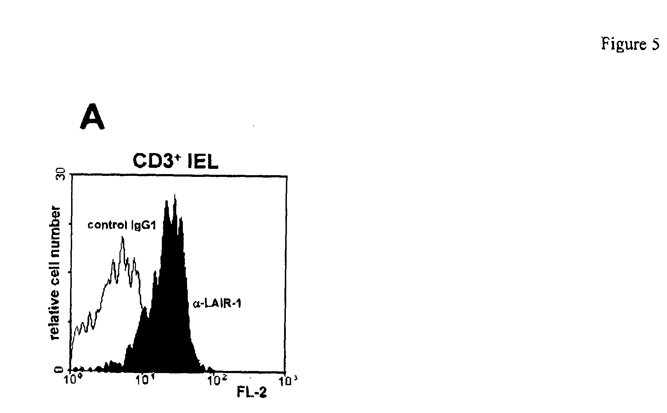

Ep-CAM is a 38 kDa transmembrane glycoprotein that is expressed on the basolateral surface of the majority of human simple epithelia (16,17). Therefore, we investigated whether LAIR-1-expressing cells in the intestine are able to interact with Ep-CAM on these epithelial cells. Isolated intraepithelial cells expressed LAIR-1 as analyzed by flow cytometry (Fig. 5 A). Moreover, tissue sections from human colon stained with anti-LAIR-1 and anti-Ep-CAM antibodies demonstrated LAIR-1-expressing intestinal intraepithelial lymphocytes in close proximity to Ep-CAM expressing epithelial cells (Fig. 5 B). This shows that these cells are subject to down-regulation through Ep-CAM/LAIR-1 interactions, which provides a mechanism to regulate mucosal immunity in the intestine.

### Figure legends.

Figure 1. LAIR-1L is identical to Ep-CAM. (A) LAIR-1-hIg binding to the colon-carcinoma cell line HT29, demonstrating the presence of a putative LAIR-1L. (B) LAIR-1-hIg binding to 293T cells transfected with the LAIR-1L cDNA obtained by expression cloning from an HT29 cDNA library. (C) Ep-CAM expression on HT29 cells and (D) Ep-CAM expression on 293T cells transfected with the LAIR-1L cDNA clone.
Filled histograms represent staining with LAIR-1-hIg (A and B) or FITC-conjugated anti-Ep-CAM mAb UBS54 (C and D). Open histogram represents staining with isotype control antibodies (A and C) or staining of 293T cells transfected with an irrelevant cDNA (B and D).

Figure 2. Anti-Ep-CAM antibodies inhibit binding of LAIR-1-hIg. HT29 cells were stained with LAIR-1-hIg or control hIg after prior incubation for 20 min at RT with 1 µg control (filled histogram) or anti-Ep-CAM 323/A3 F(ab')2 fragments (open histogram). Dashed histogram represents staining with anti-Ep-CAM and control human IgG1.

Figure 3. Both LAIR-1 and LAIR-2 bind Ep-CAM. LAIR-1-hIg (open histograms) and LAIR-2-hIg (dashed histograms) binding to the colon-carcinoma cell line HT29 (A) and to 293T cells transfected with Ep-CAM cDNA (B). Filled histograms represent staining with control human IgG1.

Figure 4. LAIR binds to the first EGF domain of Ep-CAM. A. Myc-tagged deletion constructs of human Ep-CAM (LK57-LK63) and wild type human Ep-CAM (LK17) were transfected into 293T cells and binding of LAIR-1-hIg and LAIR-2-hIg was assessed by flow cytometry. Results are indicated with ++ for strong binding and - for absence of binding. The domains of Ep-CAM are indicated with boxes (15). Amino acids (aa) are indicated with numbers, starting from the first methionine. Leader = leader peptide (aa 1-22); EGF-1 = epidermal growth factor-like domain 1 (aa 27-59); EGF-2 = epidermal growth factor-like domain 2 (aa 66-135); TM = transmembrane region (aa 267-288); myc = myc tag.
B. Representative histograms of LAIR-1-hIg binding to 293T cells transfected with the constructs indicated. Cells were stained with LAIR-1-hIg (filled histograms) or control hIg (open histograms). The middle panel represents the binding to cells transfected with constructs LK57 through LK62. Similar results were obtained with LAIR-2-hIg (data not shown).

Figure 5. LAIR-1 is expressed on human intestinal intraepithelial lymphocytes.
A. Intraepithelial lymphocytes (IEL) isolated from colon stained with anti-CD3 and gated on CD3- positive cells. Filled histogram represents staining with anti-LAIR-1 mAb and open histogram with isotype control mAb.
   B,C,D,E. Sections from human ileum were stained with anti-LAIR-1 mAb (red), anti-Ep-CAM mAb (green) and DAPI (blue) and analyzed on a fluorescence microscope. Panels C and E are higher magnifications of the areas indicated in panels B and D respectively. Intraepithelial lymphocytes are indicated with white arrows.

### References

1. Ravetch, J. V. and L. L. Lanier. 2000. Immune inhibitory receptors. *Science* 290:84-89.
2. Meyaard, L., G. J. Adema, C. Chang, E. Woollatt, G. R. Sutherland, L. L. Lanier, and J. H. Phillips. 1997. LAIR-1, a novel inhibitory receptor expressed on human mononuclear leukocytes. *Immunity* 7:283-290.
3. Meyaard, L., J. Hurenkamp, H. Clevers, L. L. Lanier, and J. H. Phillips. 1999. Leukocyte-associated Ig-like receptor-1 functions as an inhibitory receptor on cytotoxic T cells. *J. Immunol*. 162:5800-5804.
4. Van der Vuurst de Vries, A., H. Clevers, T. Logtenberg, and L. Meyaard. 1999. Leukocyte Associated Ig-like Receptor-1 (LAIR-1) is differentially expressed during human B cell differentiation and inhibits B cell receptor-mediated signaling. *Eur. J. Immunol.* In press:
5. Poggi, A., E. Tomasello, E. Ferrero, M. R. Zocchi, and L. Moretta. 1998. p40/LAIR-1 regulates the differentiation of peripheral blood precursors to dendritic cells induced by granulocyte-monocyte colony-stimulating factor. *Eur. J. Immunol.* 28:2086-2091.
6. Long, E. O. 1999. Regulation of immune responses through inhibitory receptors. *Annu. Rev. Immunol.* 17:875-804.
7. Lanier, L. L., B. C. Corliss, J. Wu, C. Leong, and J. H. Phillips. 1998. Immunoreceptor DAP12 bearing a tyrosine-based activation motif is involved in activating NK cells. *Nature* 391:703-707.
8. Lopez-Botet, M., T. Bellon, M. Llano, F. Navarro, P. Garcia, and M. De Miguel. 2000. Paired inhibitory and triggering NK cell receptors for HLA class I molecules. *Hum. Immunol.* 61:7-17.
9. Wende, H., A. Volz, and A. Ziegler. 2000. Extensive gene duplications and a large inversion characterize the human leukocyte receptor cluster. *Immunogenetics* 51:703-713.
10.Edwards, D. P., K. T. Grzyb, L. G. Dressler, R. E. Mansel, D. T. Zava, G. W. Sledge, Jr., and W. L. McGuire. 1986. Monoclonal antibody identification and characterization of a Mr 43,000 membrane glycoprotein associated with human breast cancer. *Cancer Res.* 46:1306-1317.
11.Huls, G. A., I. A. F. M. Heijnen, M. E. Cuomo, J. C. Koningsberger, L. Wiegman, E. Boel, A. Van der Vuurst de Vries, S. A. J. Loyson, W. Helfrich, G. P. van Berge Henegouwen, M. Van Meijer, J. De Kruif, and T. Logtenberg. 1999. A recombinant, fully human monoclonal antibody with antitumor activity constructed from phage-displayed antibody fragments. *Nature Biotech.* 17:276-281.
12.Aruffo, A. and B. Seed. 1987. Molecular cloning of a CD28 cDNA by a high-efficiency COS cell expression system. *Proc. Natl. Acad. Sci. USA* 84:8573-8577.
13.Lanier, L. L., C. Chang, and J. H. Phillips. 1994. Human NKR-P1A. A disulfide-linked homo-dimer of the c-type lectin superfamily expressed by a subset of NK and T lymphocytes. *J. Immunol.* 153:2417-2428.
14.Lundqvist, C., M. L. Hammarstrom, L. Athlin, and S. Hammarstrom. 1999. Isolation of functionally active intraepithelial lymphocytes and enterocytes from human small and large intestine. *J. Immunol. Methods.* 152:253-263.
15.Balzar, M., M. J. Winter, C. J. De Boer, and S. V. Litvinov. 1999. The biology of the 17-1A antigen (Ep-CAM) . *J. Mol. Med.* 77:699-712.
16.Momburg, F., G. Moldenhauer, G. J. Hammerling, and P. Moller. 1987. Immunohistochemical study of the expression of a Mr 34,000 human epithelium-specific surface glycoprotein in normal and malignant tissues. *Cancer Res.* 47:2883-2891.
17.Moldenhauer, G., F. Momburg, P. Moller, R. Schwartz, and G. J. Hammerling. 1987. Epithelium-specific surface glycoprotein of Mr 34,000 is a widely distributed human carcinoma marker. *Br. J. Cancer* 56:714-721.

## Claims

1. A method to modulate the interaction between a LAIR and its ligand, said ligand being Ep-CAM or a functional equivalent thereof, comprising providing Ep-CAM or a functional equivalent thereof, or providing an Ep-CAM antagonist.

2. A method according to claim 1, wherein said LAIR is part of an immune cell.

3. A method according to claim 1 or 2, wherein said Ep-CAM is exposed associated with a cell surface.

4. A method according to anyone of claims 1-3, wherein said immune cell comprises a NK cell, a T cell, a B cell, a monocyte, a dendritic cell and/or a thymocyte.

5. A method according to anyone of claims 1-4, comprising modulating the interaction of the first EGF-like domain of said Ep-CAM protein or functional equivalent with said LAIR.

6. Use of an Ep-CAM antagonist for modulating the LAIR-induced "silencing" of immune cells.

7. A compound capable of at least in part reducing the LAIR-induced "silencing" of immune cells.

8. A compound according to claim 7 which comprises LAIR-2 or a functional equivalent thereof.

9. A compound according to claim 8 comprising an Ep-CAM antagonist or a functional part, derivative and/or analogue thereof.

10. Use of Ep-CAM or of a functional equivalent thereof for modulating LAIR-induced silencing.

11. Use of an Ep-CAM antagonist for the preparation of a medicament.

12. Use of an Ep-CAM antagonist for the preparation of a medicament against (colorectal) Ep-CAM expressing carcinomas.

13. Use of Ep-CAM or of a functional equivalent thereof for the preparation of a medicament.

14. Use of Ep-CAM or of a functional equivalent thereof for the preparation of a medicament against an autoimmune disease.
